Europäisches Patentamt

European Patent Office

Office européen des brevets

(19)

(11) Publication number: **0 185 432**
**A2**

# EUROPEAN PATENT APPLICATION

(21) Application number: 85202097.3

(22) Date of filing: 18.12.85

(51) Int. Cl.⁴: **G01N 33/536** , C12Q 1/56 ,
//G01N33/74,G01N33/86

(30) Priority: 21.12.84 IT 2419984

(43) Date of publication of application:
25.06.86 Bulletin 86/26

(84) Designated Contracting States:
AT BE CH DE FR GB LI LU NL SE

(71) Applicant: SCLAVO S.p.A.
Via Fiorentina 1
I-53100 Siena(IT)

(72) Inventor: Paoli, Carlo
Via Imprunetana 189
I-50023 Impruneta (Florence)(IT)
Inventor: Bardelli, Fabrizio
Via Simone Martini 98
I-53100 Siena(IT)
Inventor: Rinaldi, Federica
Casa Volterrani Vagliagli
I-53019 Castelnuovo Berardenga Siena(IT)
Inventor: Tarli, Paolo
Via Val d'Aosta 19
I-53035 Monteriggioni (Siena)(IT)

(74) Representative: Roggero, Sergio et al
Ing. Barzanò & Zanardo Milano S.p.A. Via Bor-
gonuovo 10
I-20121 Milano(IT)

(54) Immunoenzymatic method for the determination of analytes, and composition suitable to the purpose.

(57) An immunoenzymatic method is disclosed for the deter-
mination of analytes using the interaction between
staphylocoagulase and prothrombin modulated by the pres-
ence of antibodies directed agains the analyte concerned.

Fig.1

## "IMMUNOENZYMATIC METHOD FOR THE DETERMINATION OF ANALYTES, AND COMPOSITION SUITABLE TO THE PURPOSE"

A continuous need exists for fast, accurate, quantitative and qualitative techniques for immunological dosages, e.g. of small-molecule proteins in biological fluids to diagnostic purposes.

In particular, dosages of competitive type are known, wherein a determined amount of immunoglobulins can bond itself both to an antigen made detectable because it is conjugated to a tracer, and to the so-called cold antigen, which is in the biological sample.

It is clear that the amount of tracer bonded to immunoglobulins is proportional to the amount of cold antigen present in the reaction mixture.

To carry out the dosage, separating by physical methods the bonded tracer from the free one is necessary. For this reason, one speaks of "heterogeneous dosages".

Examples of heterogeneous dosages are reported in U.K. Pat. 1,489,913 and U.K. Pat. 1,460,631.

In the so-called homogeneous dosages, on the contrary, is the same antibody which, by bonding itself to the antigen, determins a modulation of tracer signal, so that separating the bonded tracer from the free one is not necessary.

Examples of such dosages are reported in U.S. Patent 3,817,837.

The interaction between staphylocoagulase and prothrombin (in particular, the human one) has been known for many years (Tager, M., J. Biol. Med. 20, 487-501, 1948), and has been deeply studied (Hemker, H.C. et al., Biochim. Biophys. Acta 379, 180-188, 1975) also recently (Hendrix, H. et al., J. Biol. Chem. 258, 3637-3644, 1983).

The picture deriving from technical papers is that of an interaction between two macromolecules, that determines the appearance of biological activity. The formation of a stable complex 1:1 (on molecular base) between the two components is fast and originates an enzymatic activity measurable both by using natural substrates, as fibrinogen, and synthetic substrates (of chromogen or fluorogen types).

Neither the interaction site between the two molecules, nor the mechanism by which a biologically active complex is obtained starting from two proteins which, if isolated, are not biologically active, is known.

We have found now that the interaction between staphylocoagulase and prothrombin can be conveniently used for the determination of analytes in an immunoenzymatic system of homogeneous type.

More precisely, the capability is exploited of modulating the formation of staphylocoagulase/prothrombin complex by antibodies directed towards an analyte previously bonded to staphylocoagulase (or to the prothrombin).

This inhibiting action of the antibody is prevented (and hence enzymatic activity shall appear) when inside the medium wherein the reaction takes place suitable amounts of free analyte are present, which saturate the reactive sites of the two immunoglobulins.

It is hence a first object of the present invention an immunoenzymatic method for the determination of analytes, also in biological fluids, comprising

(A) contacting in a clear aqueous zone

(1) sample being analyzed

(2) staphylocoagulase-analyte conjugate

(3) antibody

(4) prothrombin

(B) measuring the enzymatic activity.

The method can be alternatively carried out as follows.

(A) contacting in a clear aqueous zone

(1) sample being analyzed

(2) prothrombin-analyte conjugate

(3) antibody

(4) staphylocoagulase

(B) measuring enzymatic activity.

It results evident that the analyte conjugated to staphylocoagulase (or to prothrombin) is identical to that which must determined in the sample being examined, and that the antibody is specific for the analyte to be determined.

In schemes 1, 2 and 3 which follow, the reaction mechanism is reported of our method in the case in which the staphylocoagulase-analyte conjugate is used.

When in the reaction medium no antibodies are present, the interaction occurs between staphylocoagulase-analyte conjugate (hereinunder denominated only "conjugate" for simpleness' sake) and prothrombin, with the consequent appearance of enzymatic activity.

The situation is shown in scheme 1.

In the presence of antibodies inside the reaction medium, no interaction between conjugate and prothrombin takes place, by being prevented by the bonding of the antibody on conjugate , hence no enzymatic activity is obtained.

The situation is shown in scheme 2.

In the presence of antibodies inside the reaction medium, and of analyte in the sample, the interaction takes place between conjugate and prothrombin, and hence enzymatic activity appears.

The situation is shown in scheme 3.

The analytes which can be determined according to the present invention are constituted by the substances capable of inducing specific (polyclonal or monoclonal) antibodies both directly and after suitable processing.

The enzymatic activity resulting from the staphylocoagulase-prothrombin interaction is quantitatively determined by using suitable synthetic substrates (e.g., chromogenic and fluorogenic), or is qualitatively determined by more conveniently using the natural substrate fibrinogen, and observing the possible appearance of coagulation.

The method being the object of the present Patent Application, by being a method of homogeneous type, has the advantage of methodological simplifying, by not requiring the physical separation of free conjugate from the bonded one, as it happens on the contrary in the competitive systems of heterogeneous type.

In such method as proposed, the signal is proportional to the amount of antibodies not bonded to the analyte.

Hence, by using a fixed amount of antibodies in a system wherein also the amounts of conjugate and of prothrombin is pre-established, the resulting enzymatic activity shall depend on the amount of cold analyte present in the sample being analyzed.

A second object of the present invention is a composition suitable to the determination of analytes basically constituted by:

(1) staphylocoagulase-analyte conjugate

(2) prothrombin

(3) antibody

alternatively by:

(1) staphylocoagulase

(2) prothrombin-analyte conjugate

(3) antibody

A further object of the present invention is a kit for the immunoenzymatic determination of analytes constituted by a composition comprising staphylocoagulase-analyte conjugate, prothrombin, antibodies or, alternatively, staphylocoagulase, prothrombin-analyte conjugate and antibodies.

The present invention shall be clear from the reading of the following Examples, which however do not have limitative purpose.

Example 1

Preparation of materials

A: Staphylocoagulase

The protein is obtained by purification from culture medium of a strain of Stafilococcus aureus according to the method described by Igarashi et al. (J. Biochem. 1979, 86: 1615-1618).

B: Antigen

As the antigen pregnanediol-3-alpha-glucuronide is used, obtained by starting from pregnanediol according to Samarajeeva, P. et al. (J. Steroid Biochem. 1979, 11: 1165-1171).

C: Anti-pregnanediol-3-alpha-glucuronide immunoglobulins

Antiserum is obtained by immunizing animals with bovine albumin to which by the method by Saramajeeva et al. (J. Steroid Biochem. 1979, 11 1165-1171) pregnanediol-3-alpha-glucuronide has been covalently bonded. For the analysis, the immunoglobulinic fraction obtained by fractionation of serum with ammonium sulphate and ionexchange chromatography (N. Harboe et al. Scand. J. Immunol. Suppl. 1, 1973, col. 2, pages 161-169) is used.

D: Human prothrombin

Human plasma is purified according to the method by H. Hendrix et al. (J. Biol. Chem. 1983, 258: 3637-3644).

E: Staphylocoagulase-Antigen Conjugate

Pregnanediol-3-alpha-glucuronide is covalently bonded to staphylocoagulase by following the method by Erlanger et al (J. Biol. Chem. 1957, 228: 713-727).

F: Substrate

In spectrophotometric dosage test the substrate chromogen S 2238 cod. Daniel (obtained from Ortho) at concentration 0.5 mM is used.

The enzymatic activity is evaluated by measuring O.D. (optical density) at 405 nm.

G: Diluting buffer:

50 mM Tris, 0.15 M NaCl, 0.5 mg/ml of egg albumin, pH 7.5

Example 2

Determination of enzymatic activity of Staphylocoagulase-Antigen conjugate in the absence of antibodies

Staphylocoagulase-Antigen conjugate dissolved in diluting buffer (G) is incubated with an excess of human prothrombin over 10 minutes at 37°C, and enzymatic activity is measured by evaluating the ΔO.D. at 405 nm after the addition of the substrate (F).

It has been approximately estimated that 0.5 μmol of staphilocoagulase-antigen conjugate determine an increase in absorbance at 405 nm of 18,000/min.

By comparing the enzymatic activity of the conjugate with that of free staphylocoagulase, a loss of activity of about 30% due to covalent bonding of antigen has been calculated. In any case, a concentration of 1 pmol per ml is already enough to yield a ΔO.D./minute of 0.035.

Example 3

Determination of enzymatic activity of conjugate in the presence of antibodies

After having selected a concentration of conjugate (7 pmol/ml), incubations of 10 minutes at 37°C with scalar amounts of immunoglobulins directed towards the antigen are carried out. Prothrombin is subsequently added, an after further 10 minutes the substrate is added.

An inhibition is observed of enzymatic activity proportional to the concentration of immunoglobulins.

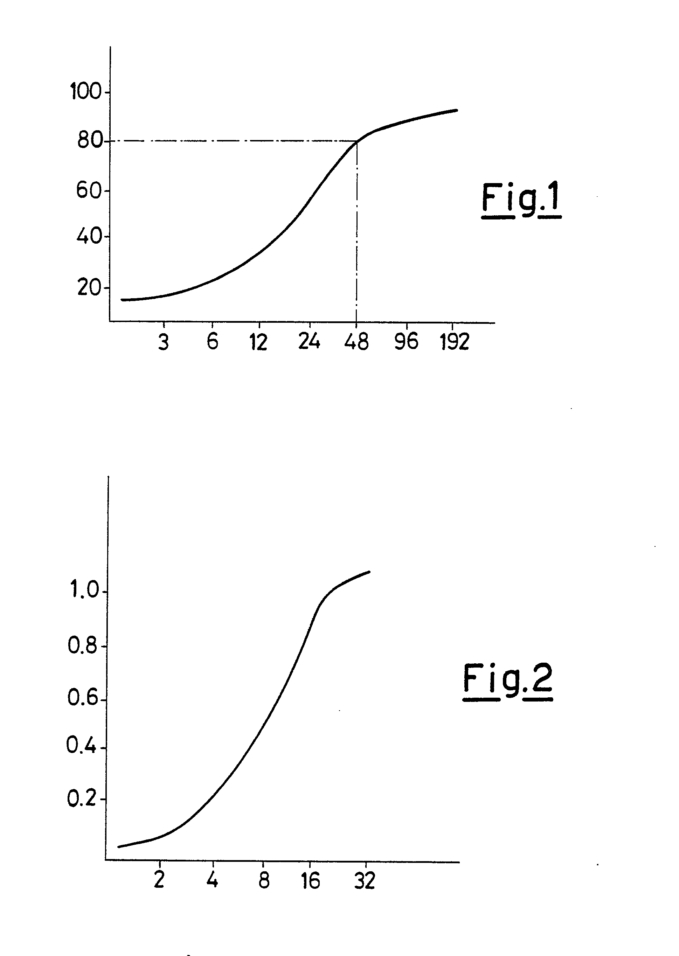

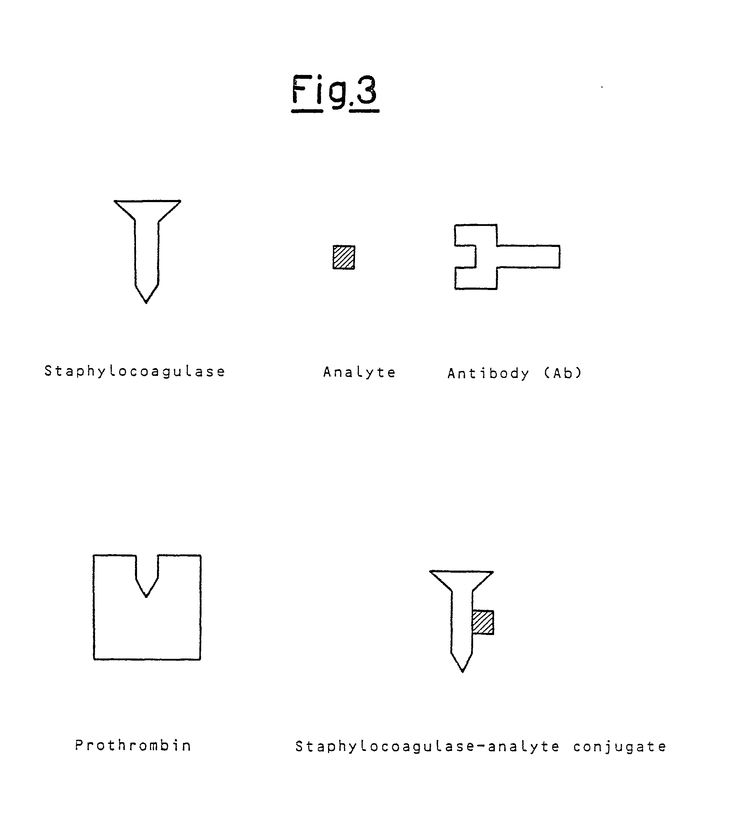

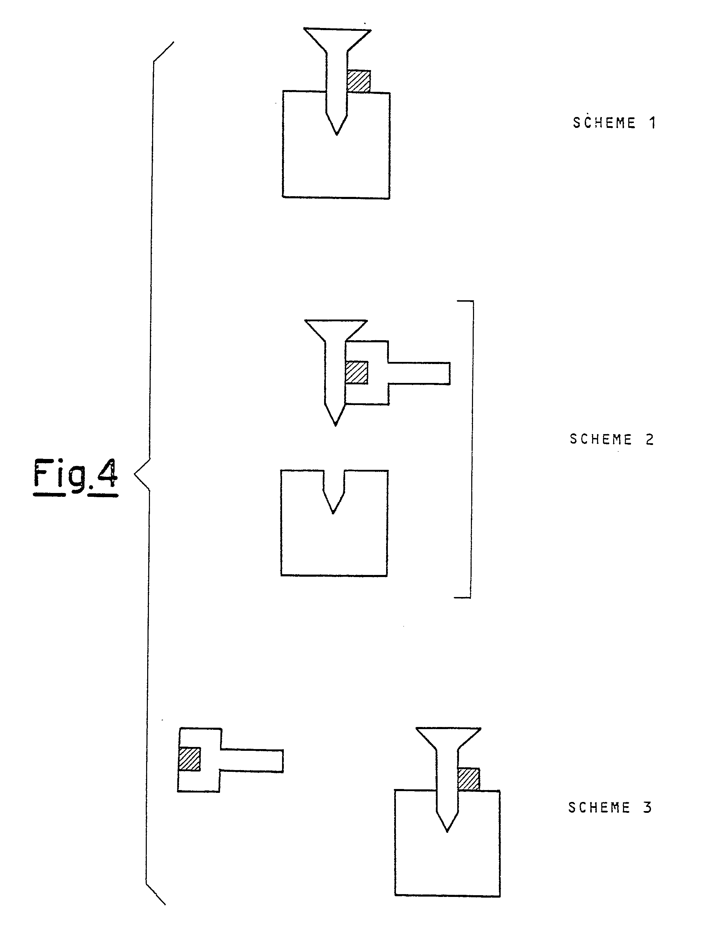

Fig. 1 reports the results of inhibition curve.

In abscissa the concentration of immunoglobulin expressed as μg/ml, and in ordinate the percent inhibition (%) is reported.

In the dosage of pregnanediol-3-alpha-glucuronide the concentration of 48 μg/ml of immunoglobulins is used, which is capable to inhibit about 80% of enzymatic activity.

Example 4

Calibration Curve

In order to obtain a reference curve, solutions are prepared of pregnanediol-3-alpha-glucuronide standard at scalar concentrations of from 2 to 32 nmol/ml.

To 0.1 ml of staphylocoagulase/pregnanediol-3-alpha-glucuronide conjugate (70 nM), 10 μl of each standard solution and 100 μl of immunoglobuline solution at the concentration of 480 μg/ml are added. After 10 minutes at 37°C, 0.1 ml of human prothrombin (100 nM) are added, and the whole is made incubate over further 10 minutes at 37°C, before adding 0.7 ml of 0.8 mM substrate solution in diluting buffer.

The difference of absorbance at 405 nm produced over 10 minutes of incubation at 37°C is determined against a reactant' blank.

The zero of obtained curve is moreover computed by adding buffer only instead of standard solution.

Fig. 2 shows a calibration curve wherein in ordinates the Δ O.D.'s relatively to the value obtained at point 0, and in abscissa the concentration of pregnanediol-3-alpha-glucuronide expressed as nmol/litre are reported.

Example 5

Determination of pregnanediol-3-alpha-glucuronide in urine samples

In order to carry out the dosage, 10 μl of urine are used in lieu of 10 μl of standard solution. A dosage in parallel relatively to reference solutions is carried out, and ΔO.D.'s values of various samples are computed by subtracting the value of reference blank from the value found.

By reporting various ΔO.D.'s on calibration curve, the concentration of pregnanediol-3-alpha-glucuronide in urine samples is obtained.

**Claims**

1. Immunoenzymatic method for the determination of analytes comprising

A) contacting in a clear aqueous zone

1) sample to be analyzed

2) staphylocoagulase-analyte conjugate

3) antibody

4) prothrombin;

B) measuring the enzymatic activity.

2. Immunoenzymatic method for the determination of analytes comprising

A) contacting in an aqueous zone

1) sample to be analyzed

2) prothrombin - analyte conjugate

3) antibody

4) staphylocoagulase;

B) measuring enzymatic activity.

3. Composition suitable to the determination of analytes basically constituted by

1) staphylocoagulase analyte conjugate

2) antibody

3) prothrombin.

4. Composition suitable to the determination of analytes basically constituted by

1) prothrombin- analyte conjugate

2) antibody

3) staphylocoagulase.

5. Kit for the immunoenzymatic determination of analytes constituted by a composition comprising staphylocoagulase - analyte conjugate, prothrombin, antibody.

6. Kit for the immunoenzymatic determination of analytes constituted by a composition comprising staphylocoagulase, prothrombin - analyte conjugate, antibody.

Fig.1

Fig.2

# Fig.3

Staphylocoagulase          Analyte      Antibody (Ab)

Prothrombin          Staphylocoagulase-analyte conjugate

SCHEME 1

SCHEME 2

Fig.4

SCHEME 3